Europäisches Patentamt

European Patent Office

Office européen des brevets

(19)

(11) Veröffentlichungsnummer: **0 160 272**
**A2**

(12)

# EUROPÄISCHE PATENTANMELDUNG

(21) Anmeldenummer: 85105062.5

(22) Anmeldetag: 25.04.85

(51) Int. Cl.⁴: **A 61 M 1/34**

---

(30) Priorität: 30.04.84 DE 3416057

(43) Veröffentlichungstag der Anmeldung:
06.11.85 Patentblatt 85/45

(84) Benannte Vertragsstaaten:
DE FR GB IT

(71) Anmelder: Fresenius AG
Gluckensteinweg 5
D-6380 Bad Homburg(DE)

(72) Erfinder: Kalepky, Ulrich, Dr.
Oberurseler Strasse 31
D-6370 Oberursel 5(DE)

(74) Vertreter: KUHNEN & WACKER Patentanwaltsbüro
Schneggstrasse 3-5 Postfach 1729
D-8050 Freising(DE)

---

(54) Hämodialysevorrichtung.

(57) Hämodialysevorrichtung, bei der die Dialysierflüssigkeit aus wenigstens einem Konzentrat mit Hilfe einer ersten und zweiten Pumpe hergestellt wird, wobie die erste Pumpe soviel Konzentrat fördert, daß eine Basisdialysierflüssigkeit eines bestimmten unteren Werts erzeugt wird, und von einem separaten Überwachungssystem überwacht wird.

Die zweite Konzentratpumpe fördert zusätliches Konzentrat in einem begrenzten Bereich und läßt sich durch eine Programmsteurereinheit steuern. Sie ist somit hardwaremäßig begrenzt und verfügt über ein eigenes Überwachungssystem.

FIG. 1

EP 0 160 272 A2

Croydon Printing Company Ltd

Patentanwälte/European Patent Attorneys
Rainer A Kuhnen*. Dipl -Ing
Paul-A Wacker*. Dipl -Ing , Dipl -Wirtsch -Ing
Wolfgang Luderschmidt**. Dr . Dipl -Chem

FRESENIUS AG

6380 Bad Homburg vdH

55 FR08 67 4

## Hämodialysevorrichtung

Die Erfindung betrifft eine Hämodialysevorrichtung mit
einem Dialysator, der zwei durch eine Membran getrennte
Kammern aufweist, von denen die eine in einen Dialysierflüssigkeitsweg und die andere in einen Blutweg geschaltet ist, mit einer im Dialysierflüssigkeitsweg angeordneten Einrichtung zur Entziehung von Ultrafiltrat, mit
einer Einrichtung zur Bereitstellung von frischer Dialysierflüssigkeit, die eine mit einer Mischkammer verbundene
Frischwasserquelle, wenigstens einen mit der Mischkammer verbundenen Konzentratbehälter,wenigstens eine erste Pumpe zur
Förderung bestimmter Konzentratmengen in die Mischkammer
und wenigstens eine zweite Einrichtung für die Förderung
zusätzlicher Konzentratmengen zur Erhöhung der Ionenkonzentration in der Dialysierflüssigkeit aufweist, sowie
mit einer Einheit zur Überwachung der Zusammensetzung der
Dialysierflüssigkeit.

Bei der Dialyse eingesetzte Blutreinigungsgeräte sollen
bekanntlich die funktionsunfähige Niere im Fall der
akuten oder chronischen Urämie ersetzen. Obwohl diese
unvollständig sind, ist es jedoch im Laufe der letzten
vier Jahrzehnte gelungen, das Verfahren soweit zu entwickeln, daß einer großen Anzahl von chronisch-urämischen

**Büro Frankfurt/Frankfurt Office:

Adenauerallee 16    Tel. 06171/300-1
D-6370 Oberursel    Telex: 526547 pawa d

*Buro München/Munich Office:

Schneggstraße 3-5    Tel 08161/6209-1
D-8050 Freising    Telex 526547 pawa d

Telegrammadresse: Pawamuc — Posischeck München 136052-802
Telefax: 08161/6209-6 (GP. 2+3) — Teletex 8161800=pawaMUC

Patienten (in Europa ca. 50.000) ein Überleben bei befriedigender Lebensqualität gesichert werden kann.

Das Blut urämischer Patienten wird dabei über eine semipermeable Membran entgiftet, wobei durch die dem Blut abgewandte Kammer des Dialysators eine Dialysierflüssigkeit geleitet wird.

Diese Dialysierflüssigkeit muß näherungsweise die gleiche Gesamtionenkonzentration aufweisen, die im Blut vorhanden ist. Treten nämlich erhebliche Abweichungen auf, so führt dies innerhalb kurzer Zeit zu einem erheblichen Unwohlbefinden bei dem Patienten, das bei stärkerer Abweichung der Zusammensetzung bis zum Tod des Patienten innerhalb von Minuten führen kann.

Typischerweise weist eine Dialysierflüssigkeit eine Natriumionenkonzentration von etwa 135 - 140 mmol/l, eine Kaliumionenkonzentration von etwa 2 mmol/l, eine Calciumionenkonzentration von etwa 1,75 mmol/l, eine Magnesiumkonzentration von etwa 0,5 mmol/l und eine Chloridkonzentration von etwa 110 mmol/l auf.

Tatsächlich differiert jedoch die Zusammensetzung der Ionenkonzentrationen im Blut vom Patienten zum Patienten, mit der Folge , daß die Zusammensetzung der Dialysierflüssigkeit nur annähernd der tatsächlichen Zusammensetzung der Ionen im Blut entspricht. Hinzu kommt, daß durch die Entfernung von Ultrafiltrat aus dem Blut und die dadurch erzeugte Störung des Gleichgewichts ständig auch die Konzentration der Ionen im Blut bzw. in den angrenden Räumen beeinflußt wird.

Es wird angestrebt, die Entfernung von Flüssigkeit und Elektrolyten, insbesondere von Natrium, so vorzunehmen, daß es zu einer möglichst geringen Veränderung des vasalen Volumens bei der Dialyse kommt.

Ist nämlich die Zusammensetzung der Dialysierflüssigkeit nur schlecht dem Patienten angepaßt, so kommt es zu dem sog. Disäquilibriumssyndrom, was zur Folge hat, daß der Patient an Erbrechen, Übelkeit, Bewußtlosigkeit u.dgl. leiden kann.

Zur Verbesserung seines Befindens wurden Methoden vorgeschlagen (vgl. ASAIO Bd.25, 1979, S.351-353), die Natriumionenkonzentration in Abhängigkeit von der Ultrafiltrationsrate bzw. der Zeit so zu variieren, daß keine nachteiligen Symptome beim Patienten mehr auftreten.

Hierzu wird die Zusammensetzung der Dialysierflüssigkeit, insbesondere die Natriumionenkonzentration, nach einem bestimmten Schema geändert, wobei üblicherweise die Pumpe zur Zuführung des Konzentrats nach einem bestimmten Programm gesteuert wird. Hierdurch wird das Mischungsverhältnis zwischen Konzentrat und Wasser verändert. Sofern nur ein Konzentrat eingesetzt wird, das sämtliche Ionenarten aufweist, werden die Konzentrationen der übrigen Kationen um weniger als 10 % bei einer Änderung der Natriumionenkonzentration von 10 mmol/l verändert, was ohne medizinische Bedeutung bleibt. Falls die Änderung der Natriumionenkonzentration von 10 mmol/l überschritten wird, so ist die Auswirkung der Veränderung bei den übrigen Kationen nicht mehr vernachlässigbar.

Die Zusammensetzung der Dialysierflüssigkeit wird dabei von einer Einrichtung, regelmäßig von einer Leitfähigkeitszelle überwacht, die bei einer Abweichung um $\pm$ 5 % vom Sollwert einen Alarm abgibt.

Es gibt Dialysevorrichtungen, bei denen die Dialysierflüssigkeit mit Hilfe von Dosierpumpen volumetrisch gemischt wird. Andererseits gibt es jedoch aber auch Geräte (DE-OS 19 18 063), bei denen die Mischung mit einem Leitfähigkeitssystem gesteuert wird, was -wie nachstehend erläutert- problematisch ist.

Kritisch ist die Sicherheit eines solchen Systems, insbesondere wenn sich die Zusammensetzung der Dialysierflüssigkeit während der Behandlungsdauer eines Patienten nach einem vorherbestimmten Programm ändert. Hierzu muß nämlich das Leitfähigkeitsfenster entsprechend der jeweiligen Zusammensetzung der Dialysierflüssigkeit verschoben werden.

Eine ausreichende Sicherheit läßt sich jedoch nur erreichen, wenn Mischung und Überwachung diversitär sind, d.h. vom Prinzip her unterschiedlich arbeiten.

Dies ist jedoch bei einer leitfähigkeitsgeregelten Mischung nicht der Fall. Als Folge kann sich die Zusammensetzung der Dialysierflüssigkeit bei einem ersten Fehler ändern, ohne daß sich die Leitfähigkeit ändert. Infolge dessen ist die bloße Verwendung von getrennten Meßzellen für die Regel- und Überwachungseinrichtung nicht ausreichend.

Besteht die Dialysierflüssigkeit weiterhin aus mehr als einer Komponente, d.h wird sie aus mehr als einem Konzentrat gemischt, so ist die Überwachung der Summenleitfähigkeit bei leitfähigkeitsgeregelten Mischsystemen im allgemeinen nicht ausreichend. Nur wenn die Beiträge der Komponenten zur elektrischen Leitfähigkeit von gleicher Größenordnung sind oder der Totalausfall einer Komponente die Sicherheit des Patienten nicht gefährdet, ist diese Methode zulässig.

Wie bereits vorstehend erläutert, bewirkt die Änderung der Summenleitfähigkeit durch Konzentrationsänderung einer Komponente prinzipiell ein erhöhtes Risiko, da die Mischeinrichtung und die Alarmgrenzen von einer Funktionseinheit gesteuert werden.

Soll die Änderung der Ionenkonzentration rechnergesteuert durchgeführt werden, so muß der Rechner sowohl das Mischsystem als auch die Überwachungseinrichtung steuern, mit der Folge, daß die Sicherheit bei einem ersten auftretenden Fehler nicht mehr gegeben ist. Dies gilt insbesondere bei solchen Geräten, bei denen die Konzentratpumpe unmittelbar durch den Rechner geregelt wird.

Die DE-OS 32 16 527 beschreibt eine Vorrichtung zur Erzeugung der Dialysierflüssigkeit, wobei die übliche Proportionierungspumpe eingesetzt wird, die in einem Verhältnis von 1:34 arbeitet. Um die Ionenkonzentration der Dialysierflüssigkeit zu senken bzw. zu erhöhen, ist in dieser Vorrichtung eine Bypass-Leitung vorgesehen, die stromauf der Proportionierungspumpe von der Wasserleitung abgeht und stromab der Proportionierungspumpe mit der Dialysierflüssigkeitsleitung verbunden ist.

In diese Bypass-Leitung ist ein Zumischventil eingeschaltet, das durch die stromab der Proportionierungspumpe in die Dialysierflüssigkeitsleitung eingeschaltete Leitfähigkeitszelle geregelt geöffnet bzw. geschlossen wird.

Das Zumischventil wird also entsprechend einer üblichen Regelungsschaltung mit Hilfe der Leitfähigkeitszelle betätigt, was die vorstehend erwähnten Probleme mit dem ersten auftretenden Fehler nach sich zieht.

Zusätzlich wird die Verbindung der Bypass-Leitung mit der Konzentratleitung vorgeschlagen mit der Folge, daß die Dialysierflüssigkeit nicht nur mit Wasser verdünnt, sondern auch mit Konzentrat zur Anreicherung der Ionenkonzentration versehen werden kann. Auch hier erfolgt wiederum die Regelung der zusätzlichen Konzentratforderung mit Hilfe der Leitfähigkeitsmeßzelle, was die bekannte Fehlerproblematik nach sich zieht.

Aus der DE-PS 32 34 119 und aus der GB-PS 20 25 787 sind Hämodialysevorrichtungen bekannt, bei denen ebenfalls zur Überwachung der Zusammensetzung der Dialysierflüssigkeit Leitfähigkeitszellen eingesetzt werden. Wie jedoch diese Zellen auf einen ersten auftretenden Fehler überwacht werden sollen, ist jedoch nicht beschrieben.

Der Erfindung liegt daher die Aufgabe zugrunde, eine Hämodialysevorrichtung der eingangs erwähnten Art zur Verfügung zu stellen, bei der die Veränderung der Ionenkonzentration der Dialysierflüssigkeit sicher rechnergesteuert auch für den Fall eines beliebigen ersten Fehlers erfolgt.

Die Lösung der Aufgabe erfolgt dadurch, daß die zweite Einrichtung eine zweite Konzentratpumpe ist, die rückkopplungsfrei durch eine Programmsteuereinheit steuerbar ist, und daß die Programmsteuereinheit mit der Einheit zur Überwachung der Zusammensetzung der Dialysierflüssigkeit verbunden ist und in dieser den Überwachungsbereich entsprechend der Förderrate der zweiten Konzentratpumpe verschiebt.

Die erfindungsgemäße Vorrichtung weist zunächst den Vorteil auf, daß sie eine Basiszusammensetzung der Dialysierflüssigkeit mit Hilfe der ersten Konzentratpumpe erzeugt, die unabhängig von einer Programmeinheit arbeitet und stets die gleiche Konzentratmenge zudosiert. Dieser ersten Konzentratpumpe ist ein unabhängiges und redundantes Sicherheitssystem zugeordnet.

Die Basiszusammensetzung der Dialysierflüssigkeit hängt somit von dem eingesetzten Konzentrat ab, wobei Basis-Natriumkonzentrationen von 125 - 150 mmol/l erreichbar sind, sofern nur ein Konzentrat eingesetzt wird.

Daneben können aber auch noch weitere Konzentrate, beispielsweise ein Natrium-Hydrogencarbonatkonzentrat, Kaliumionen-, Calciumionen- oder Magnesiumionenkonzentrat eingesetzt werden, die jweils mit einer ersten Konzentratpumpe in die Mischkammer gefördert werden.

Erfindungsgemäß kann also die zweite Konzentratpumpe einem Steuerprogramm, beispielsweise einem Programm zur Durchführung des sog. Natrium-modellings, unterzogen werden, um die Ionenkonzentration, insbesondere die Konzentration der Natriumionen, programmabhängig zu erhöhen.

Generell stellt dabei die Basiszusammensetzung der Dialysierflüssigkeit die untere Grenze der Zusammensetzung der Ionenkonzentration dar, die auch bei einem völligen Ausfall der zweiten Konzentratpumpe nicht unterschritten werden kann.

Die zweite Konzentratpumpe ist erfindungsgemäß so ausgelegt, daß sie nur einen Teil des Fördervolumens der ersten Konzentratpumpe aufweist. Sie ist somit in ihrem Fördervolumen begrenzt und fördert vorteilhafterweise höchstens eine solche Menge Konzentrat, daß zusätzlich bis zu 60 mmol/l Natriumionen zu der Dialysierflüssigkeit hinzugefördert werden. Dies gilt in ähnlicher Weise für die weiteren Ionen, d.h. die zweite Konzentratpumpe ist so ausgelegt, daß generell kein lebensbedrohender Zustand beim Ausfall der Programmmiereinheit erreicht wird. So würde ein Totalausfall der Steuerungseinrichtung allenfalls zu einer Natriumkonzentration in der Dialysierflüssigkeit von etwa 185 mmol/l führen, was lediglich zur Folge hätte, daß der Patient einen erheblichen Durst bekäme.

Erfindungsgemäß wird das Leitfähigkeitsüberwachungsfenster ($\pm$ 3 %) der redundanten Kontrolleinrichtung innerhalb fester Leitfähigkeitsgrenzen von 12 - 16 mS/cm bei bei 25° C mittels der Programmeinheit verschoben, die zugleich die zweite Konzentratpumpe korreliert steuert. Ein Totalausfall der Steuerungseinrichtung kann allenfalls bis an die obere bzw. untere feste Leitfähigkeitsgrenze führen.

Somit lassen sich also entsprechend einem vorgewählten Programm das Leitfähigkeits-Überwachungsfenster und die zweite Konzentratpumpe durch die Steuervorrichtung in entsprechender Weise verändern, ohne daß hierdurch der Schutz desPatienten beeinträchtigt wird.

Weitere Merkmale, Ausführungsformen und Beispiele sind in der nachfolgenden Beschreibung unter Bezugnahme auf die Zeichnung erläutert.

Es zeigen:

Fig. 1 eine schematische Darstellung einer Hämodialysevorrichtung mit einer Einrichtung zur Erzeugung einer Dialysierflüssigkeit im offenen System,

Fig. 2 eine schematische Darstellung einer weiteren Hämodialysevorrichtung mit einer Einrichtung zur Erzeugung einer Dialysierflüssigkeit, die getaktet arbeitet,

Fig. 3 eine Prinzipsskizze, die die Zuordnung der Programmeinheit zur ersten und zweiten Konzentratpumpe sowie zur Leitfähigkeitsüberwachungseinrichtung zeigt und

Fig. 4 eine graphische Darstellung der Zeit-Abhängigkeit von der Leitfähigkeit bei der Veränderung der Zusammensetzung der Dialysierflüssigkeit mit Hilfe der zweiten Konzentratpumpe.

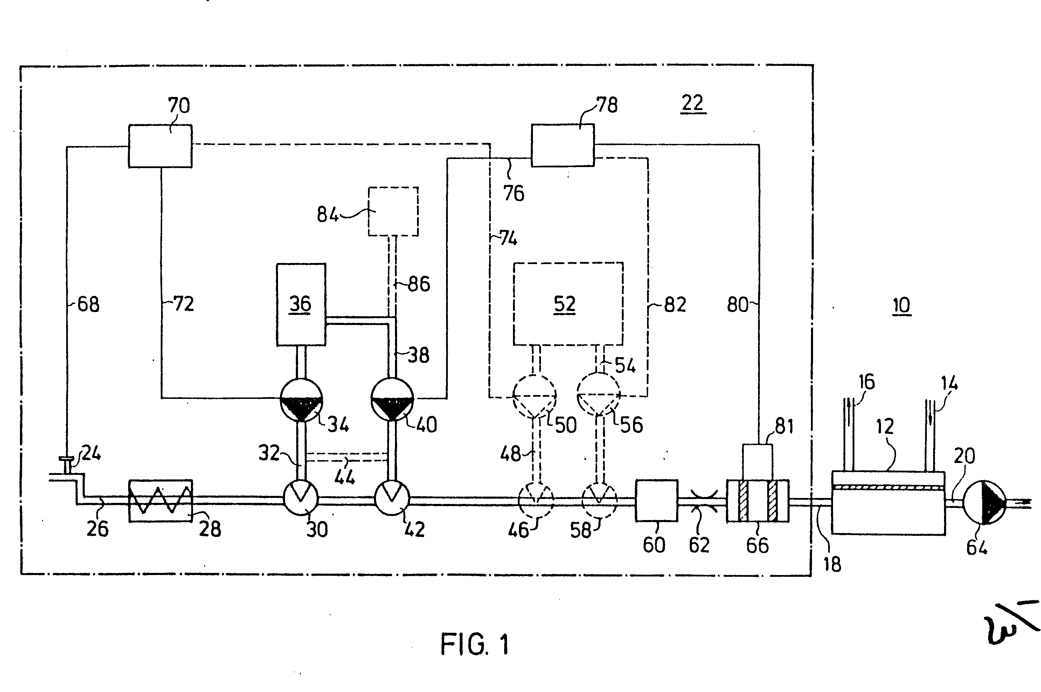

Aus Fig. 1 ist mit 10 eine Hämodialysevorrichtung ersichtlich, die einen Dialysator 12 mit Blutanschlüssen 14 und 16 sowie Dialysierflüssigkeitsanschlüssen 18 und 20 aufweist. Stromauf des Dialysierflüssigkeitsanschlusses 18 ist eine Einrichtung 22 zur Erzeugung einer bestimmten Dialysierflüssigkeit vorgesehen, die mit dem Dialysierflüssigkeitsanschluß 18 des Dialysators 12 verbunden ist.

Die Einrichtung 22 weist zunächst einen Frischwassereinlaß 24 auf, der über eine Leitung 26 mit dem Dialysierflüssigkeitsanschluß 18 verbunden ist und in die - wie nachstehend erläutert - unterschiedliche Elemente eingeschaltet sind.

Im Anschluß an den Frischwassereinlaß 24 ist eine Heizvorrichtung 28 in die Leitung 26 eingeschaltet, um das ggf. aufbereitete Wasser auf die Körpertemperatur des Patienten zu erhitzen.

An die Heizvorrichtung 28 schließt sich ein in der Leitung 26 vorgesehener Mischpunkt 30 an, der über eine Leitung 32, in die eine Pumpe 34 eingeschaltet ist, mit einem ersten Konzentratbehälter 36 verbunden ist. Dabei stellt die Pumpe 34 die erste Konzentratpumpe dar.

Von dem Konzentratbehälter 36 geht eine weitere Leitung 38 ab, in die eine zweite Pumpe 40 eingeschaltet ist, die eine zweite Konzentratpumpe darstellt. Die Leitung 38 ist mit einem weiteren Mischpunkt oder einer weiteren Mischkammer 42 verbunden, der sich gemäß der dargestellten Ausführungsform stromab des ersten Mischpunkts 30 in der Leitung 26 befindet. Dabei spielt die Anordnung dieser Mischpunkte keine wesentliche Rolle. Der Mischpunkt 42 kann auch entfallen, wobei die Leitung 38 unmittelbar mit dem ersten Mischpunkt 30 oder aber mit der Leitung 32 stromab der Pumpe 34, wie dies in Fig. 1 mit 44 gestrichelt dargestellt ist, verbunden ist.

Stromab des Mischpunkts kann gemäß einer vorteilhaften Ausführungsform ein weiterer Mischpunkt 46 vorgesehen sein, der über eine Leitung 48, in die eine Pumpe 50 eingeschaltet ist, mit einem weiteren Konzentratbehälter 52 verbunden ist.

Von diesem Konzentratbehälter zweigt wiederum eine Leitung 54 ab, in die eine Pumpe 56 eingeschaltet ist. Diese Leitung 54 ist wiederum mit einem in der Leitung 26 vorgesehenen Mischpunkt 58 verbunden. Dieser Mischpunkt 58 muß jedoch, wie bereits vorstehend festgestellt, bei dem Mischpunkt 42 nicht notwendigerweise vorgesehen sein.

Stromab des Mischpunkts 58 ist eine Entgasungseinheit 60 vorgesehen, mit der die frische Dialysierflüssigkeit entgast wird. Ein derartiges Entgasungssystem ist an sich bekannt, so daß eine weitere Beschreibung nicht erfolgt.

Stromab der Entgasungseinheit 60 ist in die Leitung 26 eine Drossel 62 eingeschaltet, die mit einer stromab des Dialysators 12 in die Leitung 26 eingeschalteten Pumpe 64, die die Dialysierflüssigkeit durch den Dialysator fördert, eine Einrichtung zur Entziehung von Ultrafiltrat darstellt. Eine derartige Einrichtung ist bei den sog. Single-Pass-Geräten, die mit einem offenen System - wie dies hier der Fall ist - arbeiten, an sich bekannt.

Weiterhin ist stromab der Mischpunkte 30, 42, 46 oder 48 ein Leitfähigkeitsmesser 66 in die Leitung 26 eingeschaltet, mit dem die Leitfähigkeit der Dialysierflüssigkeit gemessen wird, die durch die Gesamtkonzentration der in der Dialysierflüssigkeit vorhandenen Ionen bestimmt ist.

Der Frischwassereinlaß 24 ist über eine elektrische Leitung 68 mit einer Steuereinheit 70 verbunden. Die erste Konzentratpumpe 34 ist ebenfalls über eine elektrische Leitung 72 mit dieser Steuereinheit 70 verbunden.

Sofern eine Pumpe 50 in Verbindung mit einem Konzentratbehälter 52 oder aber weitere Konzentratbehälter vorgesehen sind, ist das Steuergerät 70 ebenfalls mit dieser Pumpe 50 über eine elektrische Leitung 74, die gestrichelt dargestellt ist, verbunden.

Die Wasserzufuhr durch den Frischwassereinlaß und die Konzentratzufuhr durch die Pumpe 34 wird mit Hilfe der Steuereinrichtung 70 so dimensioniert, daß ein Mischungsverhältnis von Frischwasser : Konzentrat von 34 : 1 erzielt wird. Die Zusammensetzung der Dialysierflüssigkeit hängt dabei ausschließlich von der Zusammensetzung des Konzentrats ab und ist bei vorgegebenem Konzentrat somit festgelegt.

Die Pumpe 34 ist eine übliche Konzentratpumpe, beispielsweise eine Dosierpumpe in Form einer Membranpumpe, die dem Mischpunkt exakt eine bestimmte Menge Konzentrat zuführt. Ist der Mischpunkt 30, 42, 46 oder 58 in Form einer Mischkammer ausgeführt, so kann der Mischkammer zunächst die eingestellte Konzentratmenge zugeführt werden. Anschließend erfolgt die Auffüllung der Mischkammer mit Frischwasser und zugleich die Durchmischung der in der Mischkammer enthaltenen Flüssigkeit.

Die zweite Konzentratpumpe 40 ist über eine Leitung 76 mit einer Programmsteuereinheit 78 verbunden. Diese Programmsteuereinheit 78 ist mit dem Leitfähigkeitsmesser 66 über eine elektrische Leitung 80 verbunden, in die eine Leitfähigkeitsüberwachungseinrichtung 81 eingeschaltet ist. Weiterhin kann die Programmsteuereinheit 78 mit der zweiten Konzentratpumpe 56 eines zweiten Konzentratbehälters 52 über die gestrichelte Leitung 82 verbunden sein.

Die Leitfähigkeitsüberwachungseinrichtung 81 überwacht die Zusammensetzung der Dialysierflüssigkeit mit dem von dem Leitfähigkeitsmesser 66 abgegebenen Leitfähigkeitswert, wobei sie diese Überwachung mit Hilfe eines Leitfähigkeitsüberwachungsfensters vornimmt, das bei Überschreiten eines bestimmten oberen und unteren Grenzwerts, vorzugsweise etwa $\pm$ 3 % vom vorgegebenen Soll-Wert einen Alarm abgibt bzw. die gesamte Vorrichtung abschaltet.

Die zweite Konzentratpumpe 40 bzw. 56 oder weitere Konzentratpumpen, die nicht gezeigt sind, sofern weitere Konzentratbehälter vorgesehen sind, ist vorteilhafterweise gegenüber der ersten Konzentratpumpe 34 oder 50 in ihrem Fördervolumen begrenzt und weist höchstens die Hälfte des Fördervolumens dieser ersten Konzentratpumpe 34 oder 50 auf. Die Begrenzung erfolgt hier hardwaremäßig, ist somit vorrichtungstechnisch bedingt. Dies hat den Vorteil, daß die Pumpe 40 oder 56 bei einem Ausfall der Programmsteuereinheit 78 allenfalls bis zu ihrem maximalen Förderwert hochläuft, was bei Hinzurechnung des Förderbetrags der Konzentratpumpe 34 oder 50 für einen Patienten unkritisch ist, allenfalls bei ihm also Durst erzeugt.

Die Programmsteuereinheit 78 läßt sich innerhalb der hardwaremäßig vorgegebenen Grenzen beliebig programmieren. So kann ein von der Behandlungszeit abhängiger Soll-Wert eingegeben werden, der einerseits zur Steuerung der zweiten Konzentratpumpe 40 oder 56 herangezogen wird und andererseits das Leitfähigkeitsüberwachungsmeßfenster in der Leitfähigkeitsüberwachungseinrichtung 81 verschiebt. Zu diesem Zweck ist - wie vorstehend erläutert - die Programmsteuereinrichtung 78 über die Leitung 80 mit der Leitfähigkeitsüberwachungseinrichtung 81 verbunden.

Das gesamte System ist sicher im Fall eines beliebigen ersten Fehlers, wie folgende Analyse zeigt:

| Erster Fehler | Reaktion |
|---|---|
| 1. Konzentratpumpe fällt aus | LF-Alarm = sicher |
| 2. Konzentratpumpe fällt aus | LF-Alarm = sicher |
| LF-Überwachungseinrichtung fällt aus | keine = sicher |
| Rechner steuert 2. Konzentrat-pumpe und LF-Überwachungsein-richtung synchron, aber falsch | keine = sicher, da die 2. Konzentratpumpe im sicheren Bereich festgelegt ist |

Insgesamt gesehen bietet diese Anordnung mit wenigstens zwei Pumpen für einen Konzentratbehälter eine erheblich verbesserte Sicherheit für einen Patienten als eine einzige Pumpe, die entsprechend einem von einem Rechner vorgegebenen Soll-Wert eine bestimmte Menge Konzentrat fördert.

Wie aus Fig. 1 weiterhin ersichtlich ist, kann die zweite Konzentratpumpe nicht nur mit einem Konzentratbehälter 36, sondern auch anstelle der Verbindung mit einem Konzentratbehälter, mit dem die erste Konzentratpumpe verbunden ist, mit einem weiteren Konzentratbehälter 84 über eine Leitung 86 verbunden sein. In einem solchen Fall ist die erste Pumpe 34 nur mit dem Konzentratbehälter 36 und die zweite Konzentratpumpe 40 nur mit dem Konzentratbehälter 84 verbunden. Dies kann insbesondere zweckmäßig sein, wenn außer einer Komponente, beispielsweise der Natriumkomponente, die übrige Zusammensetzung der Dialysierflüssigkeit gleich bleiben soll. So kann beispielsweise der Konzentratbehälter 84 lediglich ein NaCl-Konzentrat aufweisen, mit der Folge, daß nur die Natriumionenkonzentration in der Dialysierflüssigkeit entsprechend dem in der Programmsteuereinheit 78 vorgegebenen Programm verändert wird, während die übrigen Kationen konstant durch die Förderrate der Konzentratpumpe 34 bleiben.

Die in der Fig. 1 dargestellte Dialysevorrichtung wird auf folgende Weise betrieben:

Das Frischwasser wird durch den Frischwasseranschluß zugeführt und in der Heizvorrichtung erwärmt. Anschließend erfolgt im Mischpunkt 30 stetig oder getaktet die Zumischung eines ersten Konzentrats aus dem Konzentratbehälter 36 mit Hilfe der Konzentratpumpe 34 in einem Verhältnis von vorzugsweise 34 : 1. Die so hergestellte Dialysierflüssigkeit ist eine sog. Basisdialysierflüssigkeit, deren Ionenkonzentration unbedingt für die Behandlung des Patienten als untere Grenze erforderlich ist.

Von diesem unteren Grenzwert auf wird durch programmgesteuertes Zumischen mit der zweiten Konzentratpumpe 40 die Ionenkonzentration der Dialysierflüssigkeit angehoben und dem Patienten angepaßt.

Sofern weitere Konzentrate, beispielsweise ein Bicarbonatkonzentrat, im Konzentratbehälter 52 und ein Säurekonzentrat im Konzentratbehälter 36 bereitgehalten werden, ist eine weitere erste Konzentratpumpe 50 und eine weitere zweite Konzentratpumpe 56 vorgesehen, die in ihrer Arbeitsweise den Konzentratpumpen 34 und 40 entsprechen.

Stromab der Mischpunkte erfolgt dann die übliche Behandlung der Dialysierflüssigkeit, so die Entgasung der Dialysierflüssigkeit und die Bestimmung des Leitfähigkeitswerts.

Stromab des Leitfähigkeitsmessers 66 erfolgt dann die Zuführung der Dialysierflüssigkeit zum Dialysator und danach die Ausscheidung der gebrauchten Dialysierflüssigkeit mit Hilfe der Pumpe 64.

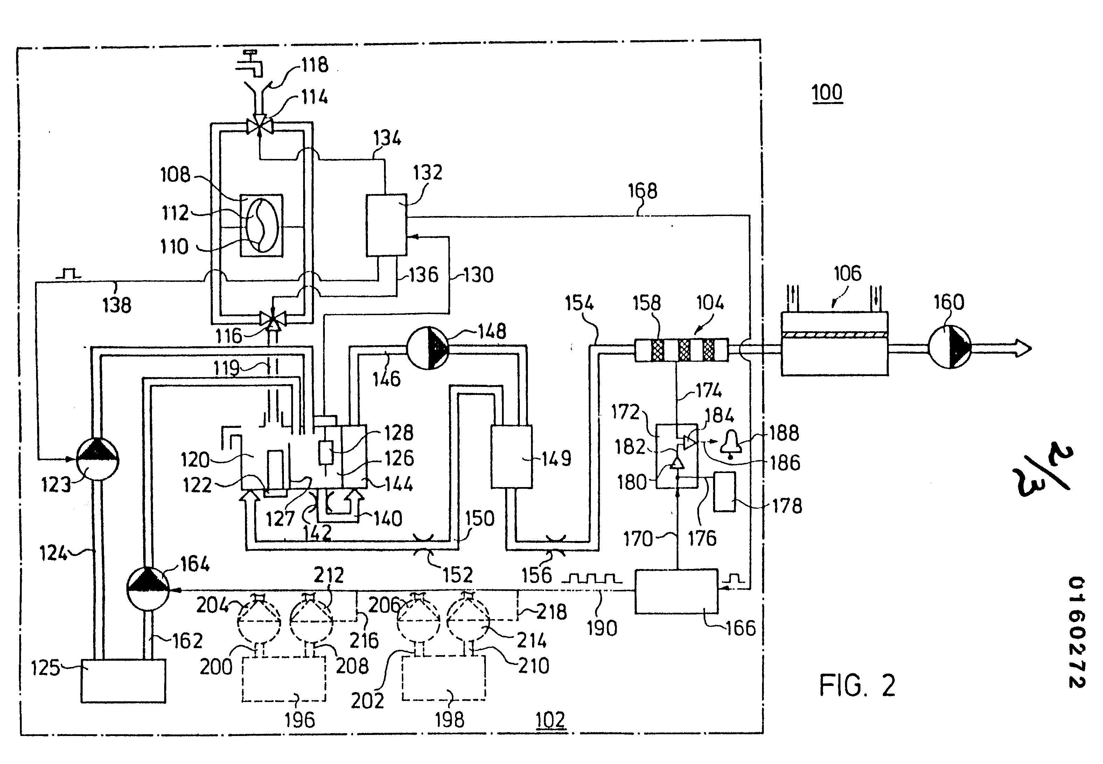

Eine weitere Hämodialysevorrichtung 100 ist in Fig. 2 gezeigt.

Sie besteht im wesentlichen aus einer Dialysierflüssigkeitszubereitungseinrichtung 102, einer Überwachungseinrichtung 104 in Form eines Leitfähigkeitsmessers und der Dialysiereinrichtung 106.

Die Dialysierflüssigkeitszubereitungseinrichtung 102 dient im wesentlichen zur Erwärmung, Entgasung und Mischung der Dialysierflüssigkeit, wobei letztere volumetrisch erfolgt.

Die Bemessung der zugeführten Wassermenge erfolgt durch die beiderseits wirkende hydraulische Pumpe 108, die aus einer durch eine elastische Membran 110 getrennte Kammer 112 und den Ventilen 114 und 116 besteht.

Wasser strömt aus dem nicht näher bezeichneten Wasseranschluß 118 unter Druck durch das Ventil 114 - was durch den Pfeil in Fig. 2 angedeutet ist - in das linke Teil der Pumpe 108 und drückt Wasser aus dem rechten Teil durch das Ventil und die Leitung 119 in die Heizkammer 120. Dort wird das Wasser durch die temperaturgeregelte Heizung 122 aufgeheizt.

Eine erste Konzentratpumpe 123 steht über eine Leitung 124 mit einem ersten Konzentratbehälter 125 in Verbindung. Sie fördert Dialysekonzentrat aus dem Konzentratbehälter 125 im Verhältnis 34 Teile Wasser : 1 Teil Konzentrat in die Mischkammer 126, die sich an die Heizkammer 120 anschließt und von dieser durch ein Überlaufwehr 127 getrennt ist.

Die Mischkammer 127 weist einen Niveaudetektor 128, beispielsweise in Form eines Schwimmerschalters auf, der das zufließende Volumen des erwärmten Frischwassers, das über das Wehr 127 strömt, begrenzt. Ist das Wasserniveau in der Mischkammer 126 um eine bestimmte Höhe gesunken, so gelangt ein Schaltimpuls über die elektrische Leitung 130 vom Niveaudetektor 128 zur Steuereinheit 132, die über die elektrischen Leitungen 134 und 136 die Ventilgruppen 114 und 116 umschaltet und über die Leitung 138 die Pumpe 123 einschaltet; damit beginnt ein Takt.

Nun strömt Wasser aus dem Wasseranschluß 118 in das rechte Teil der Membranpumpe 108 und drückt das im linken Teil der Membranpumpe 108 befindliche Wasser durch das Ventil 116 in die Heizkammer 120.

Von dort strömt das überschüssige Wasser über das Wehr 127; das Niveau steigt und der Niveaudetektor 128 wird zurückgesetzt. Nachdem der linke Teil der Membranpumpe 108 vollständig entleert ist, beginnt das Niveau zu sinken, bis der Niveauschalter erneut schaltet; damit ist ein Takt beendet.

Die Förderleistung der Konzentratpumpe auf den von der Steuereinheit 132 abgegebenen Schaltimpuls ist jedoch so groß, daß die pro Schaltimpuls in die Mischkammer 126 abzugebende Konzentratmenge in einer gegenüber der Taktzeit erheblich kürzeren Zeit zugefördert wird. Es wird also mit dieser Konzentratpumpe 123 generell sichergestellt, daß die gewünschte Basiszusammensetzung der Dialysierflüssigkeit in der Mischkammer 126 erzeugt wird.

Die Mischkammer 126 ist durch eine Leitung 140, in die eine Drossel 142 eingeschaltet ist, mit einem Unterdruckraum 144 verbunden, von dem eine Leitung 146 abgeht, in die eine Pumpe 148 eingeschaltet ist. Durch das Zusammenwirken der Drossel 142 mit der Pumpe 148 wird in dem Unterdruckraum 144 ein Unterdruck erzeugt, was die Freisetzung der in der nachströmenden Dialysierflüssigkeit enthaltenen Luft bewirkt.

Dieses Gemisch aus Luft und Dialysierflüssigkeit wird durch die Leitung 146 in ein Luftabscheidegefäß 149 gefördert, in das die Leitung 146 mündet. Die dabei freigesetzte Luft wird zusammen mit einem Teil der Dialysierflüssigkeit durch die Leitung 150 und die Drossel 152, die in die Leitung 150 eingeschaltet ist, zur Heizkammer 120, in die die Leitung 150 mündet, transportiert und entweicht dort. Die entgaste Dialysierflüssigkeit wird am Boden der Luftabscheidekammer 148 durch eine Leitung 154 abgezogen, in die ebenfalls eine Drossel 156 eingeschaltet ist. Stromab dieser Drossel 156 ist die Überwachungseinrichtung 104 in Form eines Leitfähigkeitsmessers 158 in die Leitung 154 eingeschaltet.

Stromab des Leitfähigkeitsmessers 158 mündet die Leitung 154 in den Dialysator 106, der nicht Gegenstand der Erfindung ist und deshalb nicht weiter erläutert wird. An den Dialysator 106 schließt sich wiederum eine Pumpe 160 an, die in ihrer Wirkung der Pumpe 64 gem. Fig. 1 entspricht, so daß hierauf Bezug genommen wird.

Festzustellen ist dabei, daß die erste Konzentratpumpe 123 der ersten Konzentratpumpe 34 gem. Fig. 1 entspricht, ebenso wie der Konzentratbehälter 125 dem Konzentratbehälter 36 entspricht.

Der Konzentratbehälter 125 ist gemäß der erfindungsgemäßen Ausbildung mit einer weiteren Leitung 162 verbunden, in die eine zweite Konzentratpumpe 164 eingeschaltet ist. Dabei mündet die Leitung 162 ebenso wie die Leitung 124 in der Mischkammer 126.

Die Pumpe 164 entspricht in ihrem Fördervolumen der zweiten Konzentratpumpe 40 gem. Fig. 1 und arbeitet in der gleichen Weise wie diese Pumpe, so daß hierauf Bezug genommen wird. Sie ist also in ihrem Fördervolumen hardwaremäßig begrenzt und kann somit nur eine bestimmte Konzentratmenge je Takt fördern.

Die von der zweiten Konzentratpumpe 164 zu fördernde Konzentratmenge wird in der Programmsteuereinheit 166 vorgegeben und ist dort entweder fest oder aber nach einem bestimmten Programm, beispielsweise einem Programm, das das Natrium-modelling durchführt, in Form eines Soll-Werts vorgegeben. In Fig. 2 ist dabei die Taktfolge mit 1 .... n-Takten je Arbeitstakt der Programmsteuereinheit 166 angegeben.

Diese Programmsteuereinheit 166 empfängt ihren Takt über eine elektrische Leitung 168 von der Grundsteuereinheit 132, die ebenfalls die erste Konzentratpumpe 123 synchron zum Takt steuert.

Weiterhin ist die Programmsteuereinheit 166 über eine elektrische Leitung 170 mit einer Leitfähigkeitsüberwachungseinrichtung 172 verbunden, die über eine weitere Leitung 174 mit dem Leitfähigkeitsmesser 158 verbunden ist.

Die Leitfähigkeitsüberwachungseinrichtung 172 ist über eine Leitung 176 mit einer Einrichtung 178 für die Eingabe eines Grundleitfähigkeitswerts verbunden, mit der ein bestimmter Basisleitfähigkeitswert vorzugsweise manuell,somit vorrichtungstechnisch sicher in die Leitfähigkeitsüberwachungseinrichtung 172 eingegeben werden kann. Der dabei eingegebene Leitfähigkeitswert entspricht vorteilhafterweise dem Wert der Leitfähigkeit, der der Basiszusammensetzung der von der ersten Konzentratpumpe erzeugten Dialysierflüssigkeit entspricht.

Die Leitfähigkeitsüberwachungseinrichtung 172 weist, wie in Fig. 2 dargestellt ist, vorteilhafterweise eine Summiereinheit 180 auf, die mit den Leitungen 170 und 176 verbunden ist und die Signale addiert, die von der Programmsteuereinheit 176 und der manuellen Eingabeeinheit 178 abgegeben werden. Der Ausgang der Summiereinheit 178 ist über eine elektrische Leitung 182 mit dem Eingang eines Komparators 184 verbunden, dessen weiterer Eingang mit der Leitung 174 verbunden ist, die das Signal vom Leitfähigkeitsmesser 104 in den Komparator 184 eingibt. In dem Komparator 184 werden die beiden Signale miteinander verglichen und es wird ein entsprechendes Alarmsignal am Ausgang des Komparators erzeugt, sofern die Abweichung größer als $\pm$ 3 % vom vorgegebenen Soll-Wert sein sollte. Dieser Ausgang des Komparators 184 ist über eine Leitung 186 mit einer Alarmeinrichtung 188 verbunden und betätigt diese im Alarmfall.

Weiterhin schaltet die Leitfähigkeitsüberwachungseinrichtung 172 die gesamte Vorrichtung im Alarmfalle ab.

Die Programmsteuereinheit 166 ist über eine Leitung 190 mit der zweiten Konzentratpumpe 164 verbunden und steuert diese nach Erhalt des Takts von der Grundsteuereinheit 132, wie dies vorstehend beschrieben ist. Demzufolge können je Grundsteuertakt n-Takte mit der zweiten Konzentratpumpe 164 durchgeführt werden, wobei n von 0 bis zum maximalen Leistungsvermögen reicht, das - wie vorstehend erläutert - vorrichtungstechnisch begrenzt ist.

Die zweite Konzentratpumpe 186 gibt also auf den Takt der Programmsteuereinheit 166 hin eine bestimmte, durch die Programmsteuereinheit 166 vorgegebene Menge zusätzliches Konzentrat an die Mischkammer 126 ab. Dabei entspricht diese Programmsteuereinheit 166 im übrigen der Programmsteuereinheit 78 gem. Fig. 1.

Gemäß der in Fg. 2 gezeigten Ausführungsform können weitere Konzentratbehälter 196 und 198 vorgesehen sein, die gegenüber dem Konzentratbehälter 125 unterschiedliche Konzentrate aufweisen. So kann der Konzentratbehälter 196 ein Kochsalz-Konzentrat aufweisen, während der Konzentratbehälter 198 ein Säure-Konzentrat für eine Bicarbonat-Dialyse aufweisen kann, sofern der Konzentratbehälter 125 ein Soda- oder Natriumhydrogencarbonatkonzentrat aufweist.

Andererseits sind jedoch auch andere Konzentrate, beispielsweise ein Kalium-, Calcium- oder Magnesium-Konzentrat einsetzbar.

Diese Konzentratbehälter 196 bzw.198 weisen jeweils eine erste Leitung 200 bzw.202 auf, in die eine erste Konzentratpumpe 204 und 206 eingeschaltet ist. Diese Konzentratpumpen 204 und 206 entsprechen in ihrer Wirkung der Konzentratpumpe 123. Weiterhin geht von diesen Konzentratbehältern 196 bzw.198 jeweils gemäß dieser Ausführungsform eine zweite Leitung 208 bzw.210 ab, in die die zweiten Konzentratpumpen 212 bzw.214 eingeschaltet sind.

Gemäß einer weiteren Ausführungsform entfällt die erste Konzentratpumpe 204 bzw.206 mit den entsprechenden Zuleitungen 200 bzw.202, so daß lediglich nur eine Leitung 208 bzw.210 mit den entsprechenden zweiten Konzentratpumpen 212 bzw.214 übrigbleibt. Dies ist insbesondere dann sinnvoll, wenn ein in dem Behälter 125 vorliegendes Grundkonzentrat vorhanden ist und lediglich auf die Basiszusammensetzung eine weitere Zusammensetzung aufgesetzt werden soll. In einem solchen Fall kann im übrigen auch die zweite Konzentratpumpe 164 des ersten Konzentratbehälters 125 entfallen, d.h. der erste Konzentratbehälter 125 liefert die für die Basiszusammensetzung nötige Konzentratmenge, während die zweiten Konzentratpumpen 212 oder 214 eine weitere begrenzte Menge eines anderen Konzentrats liefern.

Hinzuzufügen ist, daß natürlich die Leitungen 200, 202, 208 und 210 jeweils in der Mischkammer 126 münden, was aus Übersichtlichkeitsgründen jedoch in der Fig. 2 nicht dargestellt ist. Weiterhin sind die zweiten Konzentratpumpen natürlich über elektrische Leitungen 216 und 218 mit der Steuereinrichtung 166 verbunden und werden durch diese gesteuert. In ähnlicher Weise sind die ersten Konzentratpumpen 204 und 206 durch nicht gezeigte elektrische Leitungen mit der Steuereinrichtung 132 verbunden, die der Leitung 138 entsprechen.

Wie bereits vorstehend erwähnt, können die zweiten Konzentratpumpen 40, 56, 164, 212 oder 214 taktgesteuerte Membranpumpen sein, die volumetrisch bestimmte Mengen abgeben. Sie können jedoch aber auch Kolbenpumpen oder schrittmotorgesteuerte Schlauchpumpen sein. Diese diskontinuierlich arbeitenden Pumpen eignen sich insbesondere für die Ausführungsform gemäß Fig. 2 und gemäß Fig.1, sofern ein diskontinuierlich betriebenes Mischsystem eingesetzt wird.

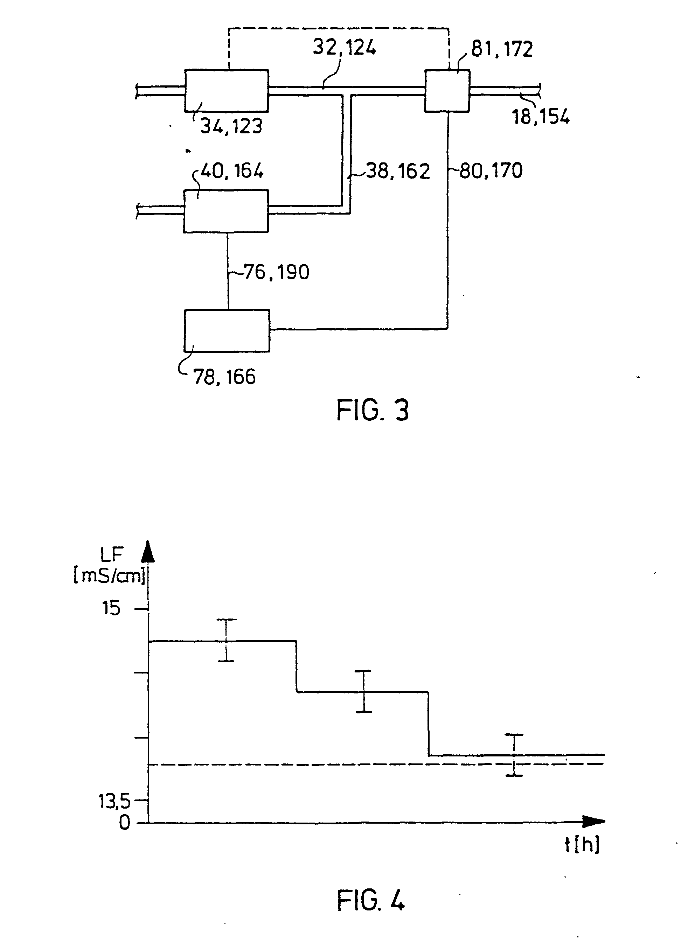

In Fig. 3 ist das wesentliche Prinzip der in Fig. 1 und 2 dargestellten Vorrichtungen erläutert. Hieraus ist ersichtlich, daß die erste Konzentratpumpe 34, 123, nicht mit der Leitfähigkeitsüberwachungseinrichtung 81, 172 verbunden ist. Ihr Grundwert wird vorteilhafterweise manuell, beispielsweise durch die Einrichtung 178, in die Leitfähigkeitsüberwachungseinrichtung 81, 172 eingegeben.

Dagegen ist die zweite Konzentratpumpe 40, 164 ebenso direkt mit der Programmsteuereinrichtung 78, 166 verbunden, wie die Leitfähigkeitsüberwachungseinrichtung 81, 172. Infolge der hardwaremäßigen Begrenzung, die durch die leistungsmäßige Begrenzung der zweiten Konzentratpumpe 40, 164 oder aber durch den gesamten Leitfähigkeitsausschnitt der Vorrichtung selbst vorgegeben ist, ergibt sich die gewünschte Sicherheit gegen einen beliebigen ersten Fehler bei dieser programmgesteuerten Einrichtung zur Erzeugung einer veränderbaren Zusammensetzung der Dialysierflüssigkeit.

In Fig. 4 ist graphisch die Veränderung der Zusammensetzung der Dialysierflüssigkeit in Abhängigkeit von der Zeit gezeigt. Dabei bedeutet die gestrichelte waagerechte Linie die Basiszusammensetzung, die durch die erste Konzentratpumpe erzeugt wird. Die treppenartige Kurve ergibt sich dann durch die entsprechende Betätigung der zweiten Konzentratpumpe, wobei dieser Anteil auf die Basiszusammensetzung aufgesetzt wird. Die treppenartige Kurve selbst liegt als Soll-Wert in zeitlicher Abfolge in der Programmeinheit 166 vor, die die zweite Konzentratpumpe 40, 164 sowie weitere zweite Konzentratpumpen entsprechend ansteuert.

Weiterhin ist aus Fig. 4 das Leitfähigkeitsüberwachungsfenster in der treppenartigen Kurve gezeigt, das in entsprechender Weise in der Leitfähigkeitsüberwachungseinrichtung 81, 172 durch die Programmsteuereinheit 78, 166 verschoben wird.

Die Leitfähigkeitsüberwachungseinrichtung 81, 172 stellt erfindungsgemäß eine bevorzugte Ausführungsform einer diversitär, redundanten Überwachungseinrichtung dar, in der in entsprechender Weise durch die Programmsteuereinheit 78, 166 der Überwachungsbereich verschoben wird. Dabei entspricht die Verschiebung dieses Bereiches der durch die zweite Konzentratpumpe erzeugten Veränderung der Zusammensetzung der Dialysierflüssigkeit, wobei der Basiswert, wie vorstehend erläutert ist, getrennt hiervon eingegeben wird.

Neben Leitfähigkeitsmessern sind als diversitär, redundante Überwachungseinrichtungen Durchflußmesser, volumetrisch arbeitende Systeme und dgl. einsetzbar. Diese diversitär redundante Überwachungseinrichtung der zweiten Konzentratpumpe ist im übrigen von derjenigen der ersten Konzentratpumpe unabhängig, also an diese nicht gekoppelt.

Neuer Hauptanspruch

1. Hämodialysevorrichtung mit einem Dialysator, der zwei durch eine Membran getrennte Kammern aufweist, von denen die eine in einen Dialysierflüssigkeitsweg und die andere in einen Blutweg geschaltet ist, mit einer im Dialysierflüssigkeitsweg angeordneten Einrichtung zur Entziehung von Ultrafiltrat, mit einer Einrichtung zur Bereitstellung von frischer Dialysierflüssigkeit, die eine mit einer Mischkammer verbundene Frischwasserquelle, wenigstens einen mit der Mischkammer verbundenen Konzentratsbehälter, wenigstens eine erste Pumpe zur Förderung bestimmter Konzentratmengen in die Mischkammer und wenigstens eine zweite Einrichtung für die Förderung zusätzlicher Konzentratmengen zur Erhöhung der Ionenkonzentration in der Dialysierflüssigkeit aufweist, sowie mit einer Einheit zur Überwachung der Zusammensetzung der Dialysierflüssigkeit, d a d u r c h   g e k e n n - z e i c h n e t ,  daß die zweite Einrichtung eine zweite Konzentratpumpe (40, 56, 164, 212, 214) ist, die rückkopplungsfrei durch eine Programmsteuereinheit (78, 166) steuerbar ist, und daß die Programmsteuereinheit (78, 166) mit der Einheit (81, 172) zur Überwachung der Zusammensetzung der Dialysierflüssigkeit verbunden ist und in dieser den Überwachungsbereich entsprechend der Förderrate der zweiten Konzentratpumpe (40, 56, 164, 212, 214) verschiebt.

2. Vorrichtung nach Anspruch 1, d a d u r c h   g e k e n n - z e i c h n e t ,  daß die Einheit zur Überwachung der Zusammensetzung der Dialysierflüssigkeit eine Leitfähigkeitsüberwachungseinrichtung (81, 172) ist.

3. Vorrichtung nach Anspruch 1, d a d u r c h   g e - k e n n z e i c h n e t, daß die zweite Konzentrat- pumpe (40, 56, 164, 212, 214) nur zur Förderung einer solchen Konzentratmenge befähigt ist, die die Sicherheit des Patienten nicht gefährdet.

4. Vorrichtung nach Anspruch 1, d a d u r c h   g e - k e n n z e i c h n e t, daß die erste Konzentrat- pumpe (34, 50, 123, 204, 206) durch die Grundsteuereinheit (70, 132) getaktet gesteuert ist, die den Takt der Programmsteuereinheit (78, 166) bestimmt.

5. Vorrichtung nach Anspruch 4, d a d u r c h   g e - k e n n z e i c h n e t, daß die Programmsteuerein- heit (78, 166) n-Takte je von der Grundsteuereinheit (70, 132) vorgegebenem Takt an die zweite Konzentratpumpe (40, 56, 164, 212, 214) abgibt.

6. Vorrichtung nach Anspruch 1, d a d u r c h   g e - k e n n z e i c h n e t, daß mehrere erste Konzentrat- pumpen (34, 50, 123, 204, 206) und/oder zweite Konzentratpumpen (40, 56, 164, 212, 214) vorgesehen sind, die jeweils mit den Konzentratbehältern (36, 52 125, 196, 198) verbunden sind.

7. Vorrichtung nach Anspruch 1, d a d u r c h   g e - k e n n z e i c h n e t, daß die Programmsteuerein- heit (78, 166) die Zusammensetzung der Dialysierflüssigkeit nach einem vorgewählten Programm oder nach einem vorgegebenen konstanten Wert einstellt oder anhand eines vorgegebenen Sollwerts regelt.

8. Vorrichtung nach Anspruch 2, d a d u r c h   g e - k e n n z e i c h n e t, daß die Leitfähigkeitsüber- wachungseinrichtung (81, 172) einen Summierer, der jeweils ein Eingangssignal von der Programmeinheit (166) und einer Einheit (178) zur manuellen Eingabe eines Grundwerts empfängt, und einen Komparator (184) aufweist, der das Summensignal des Addierers (180) und das Signal des Leitfähigkeitsmessers (158) aufnimmt und diese miteinander vergleicht.

9. Vorrichtung nach Anspruch 8, d a d u r c h   g e - k e n n z e i c h n e t,   daß die Leitfähigkeits- überwachungseinrichtung (172) mit einer Alarmeinheit (188) verbunden ist.

FIG. 1

FIG. 2

FIG. 3

FIG. 4